(19) Europäisches Patentamt
European Patent Office
Office européen des brevets

(11) Numéro de publication: **0 300 849 B1**

(12) **FASCICULE DE BREVET EUROPEEN**

(45) Date de publication de fascicule du brevet: **16.06.93**

(51) Int. Cl.$^5$: **C12N 9/50**, C12N 9/08, C12N 9/96

(21) Numéro de dépôt: **88401579.3**

(22) Date de dépôt: **23.06.88**

# (54) Procédé de modification chimique de protides et produits ainsi modifiés.

(30) Priorité: **30.06.87 FR 8709198**
**25.05.88 FR 8806914**

(43) Date de publication de la demande:
**25.01.89 Bulletin 89/04**

(45) Mention de la délivrance du brevet:
**16.06.93 Bulletin 93/24**

(84) Etats contractants désignés:
**AT BE CH DE ES GB GR IT LI LU NL SE**

(56) Documents cités:
**EP-A- 0 199 644**
**FR-E- 39 628**
**US-A- 2 567 747**
**US-A- 3 940 420**

(73) Titulaire: **SOCIETE NATIONALE ELF AOUITAINE**
**Tour Elf, 2, Place de la Coupole, La Défense 6**
**F-92400 Courbevoie(FR)**

(72) Inventeur: **Levesque, Guy**
**19 rue de l'Unité,Epron**
**F-14610 Thaon(FR)**
Inventeur: **Souppe, Jérôme**
**3 rue de Babsalle**
**F-64000 Pau(FR)**
Inventeur: **Seris, Jean-Louis**
**Chemin Vignats**
**F-64110 Jurançon(FR)**
Inventeur: **Bellenger, Valérie**
**39 rue de la Pigacière**
**F-14000 Caen(FR)**

(74) Mandataire: **Bertrand, Didier et al**
**Cabinet FEDIT-LORIOT 38, Avenue Hoche**
**F-75008 Paris (FR)**

EP 0 300 849 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne un nouveau procédé pour la modification chimique de protides. Elle comprend une réaction permettant de fixer à la molécule du protide un ou plusieurs groupements organiques, susceptibles de changer les propriétés physico-chimiques de ce protide. L'invention vise, notamment, la modification de protéines et, en particulier, celle d'enzymes. Une application importante de l'invention réside dans le changement de la solubilité de peptides ou protéines et, spécialement, dans le fait de rendre des enzymes solubles dans des solvants organiques.

L'invention comprend également les protides portant des groupements organiques, modificateurs de leurs propriétés physico-chimiques. Elle vise en particulier les enzymes rendues solubles dans des solvants organiques, par la fixation desdits groupements.

Il est connu de changer plus ou moins les propriétés d'une protéine par l'action d'un réactif chimique. Dans les cas d'enzymes, cela a généralement pour but de modifier leurs propriétés physiques, telles la solubilité, la mobilité, c'est-à-dire masse moléculaire,la stabilité, etc. On cherche également, par ce moyen,de rendre possible l'utilisation de ces catalyseurs remarquables dans des conditions moins drastiques que celles qu'exige l'enzyme native,notamment les pH, température, force ionique, résistance aux inhibiteurs, etc. D'autres objectifs résident dans la modification des propriétés catalytiques elles-mêmes, c'est-à-dire dans l'élargissement de l'éventail de substrats justiciables de l'enzyme donnée, ou bien - au contraire -dans l'orientation de l'activité catalytique dans le sens d'une plus grande spécificité ; il peut s'agir par exemple de rendre une protéase plus spécifique d'une certaine liaison peptidique, si elle hydrolyse, à l'état natif, un spectre trop large de peptides ou de l'emploi d'enzymes en milieu non aqueux ou faiblement concentrés en eau qui est susceptible d'inverser le sens des réactions dans lesquelles l'eau intervient.

La plupart des procédés connus de modification chimique de protéines consistent à mettre en oeuvre des composés spécifiques, capables de réagir avec des groupements fonctionnels, portés par les résidus des acides aminés naturels de la chaîne peptidique ou par la partie glucidique ou nucléique de glycoprotéines ou de nucléoprotéines. Ainsi utilise-t-on très couramment le pouvoir nucléophile du groupement $\epsilon$ -$NH_2$ des résidus de lysine sur des réactifs électrophiles activés. Les réactifs le plus souvent utilisés, dans la technique antérieure, sont les anhydrides d'acides et l'acide trinitro benzène sulfonique. Les enzymes, modifiées par ces procédés connus, ont en général une activité sensiblement abaissée. Le choix des réactifs est très restreint, car beaucoup de réactifs éventuels altèrent trop l'activité de l'enzyme, ou bien exigent des conditions opératoires incompatibles avec la conservation de l'activité catalytique ou réagissent avec d'autres acides aminés essentiels au maintien de la structure active de l'enzyme.

Il en résulte, qu'étant donné l'intérêt de la modification appropriée de protides, en particulier d'enzymes, le besoin de réactifs adéquats continue à se faire sentir.

La présente invention apporte dans ce domaine un progrès sensible ; elle porte sur une classe de composés qui ont la propriété de réagir aisément avec des restes aminés de protides pour fixer sur ceux-ci tout groupe organique voulu, en n'abaissant pas ou relativement peu l'activité catalytique, lorsque le protide est une enzyme, et en le rendant soluble dans un solvant organique.

Le procédé de l'invention est caractérisé en ce que l'on fait réagir le protide donné avec un acide, sel ou ester dithioïque dont le reste d'acide comporte le groupe organique que l'on désire fixer au protide, tandis que le reste de la molécule a une structure de groupe partant.

L'ester, l'acide ou sel dithioïque employé peut être représenté par la formule

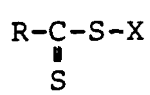 ............. (1)

où R désigne le groupe organique à fixer,par l'intermédiaire de C=S, sur le protide, alors que X est H ou un cation ou groupement choisi de telle manière que X-S soit un bon groupe partant.

En désignant par E la molécule de ce dernier, on peut schématiser comme suit la réaction suivant l'invention :

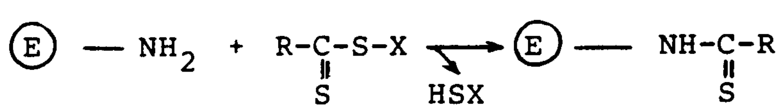 ....(2)

Ainsi, HSX étant éliminé, la molécule de protide est modifiée par la fixation d'un ou de plusieurs groupes

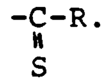

Ce groupe modificateur est choisi, naturellement, selon les nouvelles propriétés que l'on désire faire acquérir au protide, par exemple solubilité dans des hydrocarbures, réactivité avec des bases, résistance à la chaleur, etc. Il peut donc comprendre un R qui est un radical hydrocarboné, aliphatique ou arylique, donc hydrophobe, en particulier alkyle en $C_1$ à $C_{20}$ ou alcényle en $C_2$ à $C_{20}$, phényle, phénylényle, naphtyle ou naphtylényle, ou bien un alkylaryle ou aryl-alkyle correspondant, R peut également être un groupe organique hydrophile, polaire, négatif, positif ou neutre : il peut, par exemple, comporter un groupe carboxyle, hydroxyle, halogène, sulfinyle, sulfonyle, phoshoryle, phosphonyle, sulfhydryle, amide, ammonium, phosphonium, etc.

Dans une forme d'exécution particulière, R est une chaîne polymère ou oligomère, par exemple de polyéthylène, polypropylène, polyacrylique, polyéthylène glycol ou similaire.

En outre, R peut être constitué, lui-même, par un reste de dithioester du type

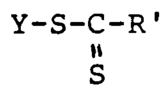

dans lequel R' désigne un groupe hydrocarboné divalent, notamment alkylène, cycloalkylène, arylène ou alkyl-arylène, le premier comportant de préférence 1 à 20 atomes de C, les autres 6 à 16. Y est un groupement du même type que X mais non forcément identique à celui-ci. Dans cette variante, le réactif est un bis-dithioester :

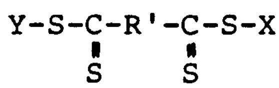 ....................... (3)

L'utilisation d'un bis-dithioester permet de former un protide modifié, spécial,porteur d'une chaîne polymère, en particulier polyacrylique, d'alcool polyvinylique, polyalkylène-glycol ou autre oligomère ou polymère susceptible d'être aminé au moins en bout de chaîne. En désignant par $Q\text{-}NH_2$ un tel polymère aminé, on peut le combiner au bis-dithioester par une réaction

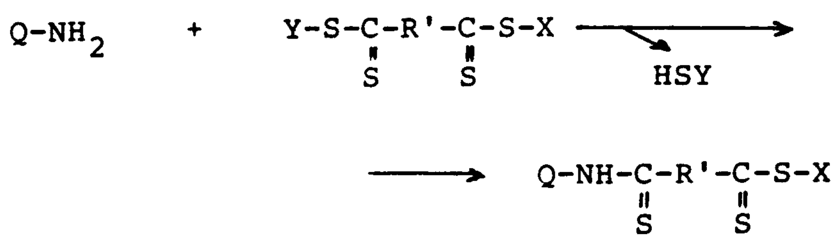


... (4)

Ensuite, en faisant réagir le composé de Q obtenu en (4) avec un protide Ⓔ-$NH_2$, on aboutit à :

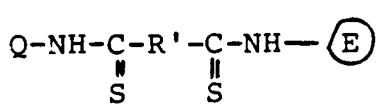 .................. (5)

c'est-à-dire un bis-thioamide à la fois du polymère Q et du protide E.

A titre d'illustration non limitative, on donne ci-après les formules de quelques uns des dithioesters et bis-dithioesters utilisables dans la réalisation de la présente invention.

$n-C_{12}H_{25}-CS_2-CH_2-COOH$

$HOOC-CH_2-S-CH_2CH_2-CS_2-C_2H_5$

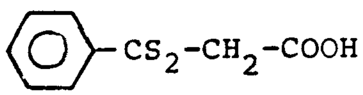

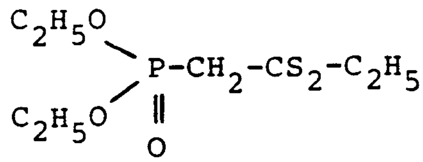

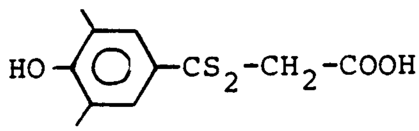

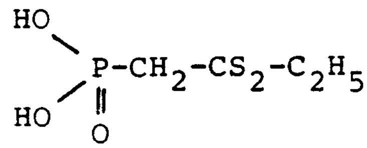

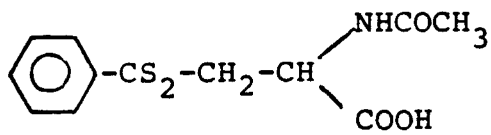

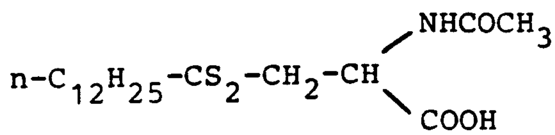

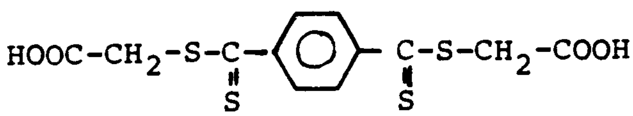

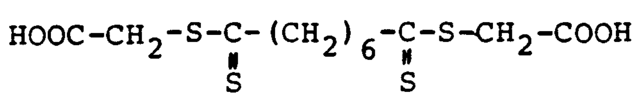

Selon la nature du protide à traiter et celle du dithioester employé, le procédé suivant l'invention est réalisé en solution aqueuse, organique ou hydro-organique, généralement à une température comprise entre 0° et 70°C. Dans les cas des enzymes les températures préférées s'échelonnent de 10° à 50°C et surtout entre 20° et 45°C.

Les proportions des réactifs sont calculées à partir du nombre de groupes -NH- ou $-NH_2$ du protide qu'il s'agit de combiner avec le dithioester. Il est en général bon d'utiliser un excès de fonctions dithioesters par groupe -NH-ou $-NH_2$ libre du protide sur lequel on veut fixer le groupe modificateur. Le milieu réactionnel peut éventuellement être hétérogène, pouvant par exemple être constitué par une dispersion du protide dans une solution organique du dithioester ; c'est en particulier le cas où la réaction suivant l'invention solubilise le protide dans le solvant organique du dithioester ; ainsi, le protide, ayant subi la réaction, passe en solution dans ce solvant.

Dans une variante, X ou/et Y, semblables ou différents, sont constitués par une partie hydrocarbonée $(-CH_2)_n$, où n est 1 à 6, portant une fonction halogène, carboxyle, sulfinique, sulfonique, phosphoreuse, phosphoneuse, phosphorique, phosphonique, H actif, SH, amide ou hydroxy.

Un protide suivant l'invention, en particulier protéase ou peroxydase, soluble dans du benzène, toluène, éther, éthanol, chloroforme ou/et diméthyl sulfoxyde, comprend plusieurs motifs lysine qui portent, par

l'intermédiaire d'un

$$-\underset{|}{C}=S-,$$

un groupe R constitué par un radical aliphatique en $C_1$ à $C_{20}$, un radical arylique en $C_6$ à $C_{16}$ ou une chaîne polymère, en particulier de polyéthylène glycol.

Lorsque X, dans la formule (1), est un atome d'hydrogène, c'est-à-dire que le composé dithioïque est un dithioacide R-CSSH, R peut être un des radicaux définis plus haut. Conviennent particulièrement bien à la réalisation de l'invention les acides R-CSSH et leurs sels d'amine ou d'ammonium quaternaire, dont le groupe R est un alkyle en $C_4$ à $C_{18}$, comme par exemple n-$C_4H_9$ ou n-$C_{11}H_{23}$-, un phényle ou tolyle -$C_6H_5$, $CH_3$-$C_6H_4$ etc.

Les sels d'acides dithioïques, convenant à l'invention, ont un cation provenant d'une base faible. La base faible, dont le sel de dithioacide R-CSS-X est utilisable suivant l'invention, peut être telle qu'ammoniaque, hydrazine, hydroxylamine, pipéridine, amine, di- ou tri-amine, primaire, secondaire ou tertiaire, pyridine, tétra-alkyl ammonium, di- ou tri-alkyl respectivement di- ou mono aryl ammonium, hydroxyde de Zn, Al, Mn, Pb, etc,et - d'une façon générale - une base dont la constante de dissociation ionique dans l'eau, à la température ordinaire, est de préférence de l'ordre de $10^{-10}$ à $10^{-3}$ et surtout de $10^{-6}$ à $10^{-4}$. Ainsi peut-on utiliser, à titre d'exemples non limitatifs, des sels dont le X est le cation de l'ammonium, de tétra méthyl-ammonium, phényl-triéthyl ammonium, triméthyl-hexadécyl ammonium, mono-éthanolamine, di-éthanol amine, dipropylamine, di-isopropylamine, pipérazine, tri-isobutyl amine, triéthylamine, benzylamine, phényléthyl amines, etc.

Le greffage suivant l'invention est effectué par la mise en contact du protide choisi avec le composé dithioïque, au sein d'une solution tampon de pH approprié. Il est bon d'employer un large excès de ce composé par rapport au protide, de préférence de 10 à 100 équivalents de R-C-S-S-X par groupe $NH_2$ disponible dans le protide. Cet excès est éliminé de la solution finale, notamment par dialyse. Il convient de prolonger ce contact durant quelques heures, le plus souvent et selon la température et la nature des réactifs en présence, pendant 1 à 20 heures, bien que cette indication ne soit nullement limitative. Bien entendu, compte doit être tenu de la stabilité du protide dans les conditions opératoires, pour ne pas prolonger la réaction au-delà du temps de stabilité du produit.

D'une façon générale, le procédé de l'invention peut être mis en oeuvre dans des milieux de pH 3 à 11,5 et surtout de 4 à 9. Cependant, il existe des domaines préférés, selon la nature du protide traité et celle du composé dithioïque utilisé. Ainsi est-il recommandable d'opérer avec un pH ne dépassant pas 7, lorsqu'on travaille sur des protides qui supportent mal le milieu basique. D'autre part, de bons résultats sont obtenus avec des composés dithioïques R-CSSX constitués par un dithioester lorsque le pH est de 7 à 11,5 et surtout pH 8 à 11. Par contre, quand X est H ou un cation de base faible, les pH préférés se rangent entre 3 et 7, ou mieux de 3,5 à 6,5 avec, en général, un optimum pour 4 à 6.

Les exemples qui suivent apportent, sans aucune limitation, des détails à la mise en oeuvre de l'invention.

## EXEMPLE 1

Les opérations décrites ici servent à montrer (I) qu'il est possible de préparer un dithioester à partir d'un autre, par simple changement de nucléofuge, et - ensuite -(II) comment le -$NH_2$ d'un amino-acide réagit avec un dithio-ester.

### I. Préparation de S-dithiobenzoyl N-acétyl cystéine (DTAC)

A 1,632 g de N-acétyl cystéine et 2,123 g d'acide thiobenzoyl mercapto acétique (ou dithiobenzoate de carboxyméthyle), dissous dans 50 ml d'éthanol, on ajoute 2 ml de solution aqueuse de NaOH M. Le mélange est agité à 20°C, pendant 2 heures et ensuite concentré, par évaporation d'éthanol, et acidifié avec de l'HCl 6N.

Par chromatographie sur silice, avec comme éluant un mélange hexane/chloroforme, 2,45 g de S-thiobenzoyl N-acétyl cystéine sont isolés, ce qui représente un rendement de 86,5%, par rapport à l'acétyl cystéine de départ, de la réaction :

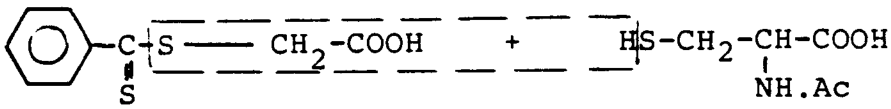

Acide thiobenzoyl mercaptoacétique

Acétyl cystéine

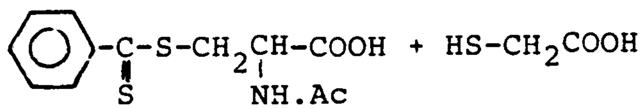

S-dithiobenzoyl N-acétyl cystéine (DTAC)

Acide mercapto acétique

II. Préparation de la N-thiobenzoyl lysine à partir de dithiobenzoyl acétyl-cystéine (DTAC)

0,425 g soit 1,5 mmole de DTAC, préparée suivant I, et 0,220 g de lysine, également 1,5 mmole, sont dissous dans 10 ml de solution aqueuse de NaOH 0,25 N. La solubilisation du mélange est facilitée par l'addition d'une goutte d'éthanol. Après 5 heures de réaction à la température ambiante, le milieu est acidifié et libère la N-thiobenzoyl lysine

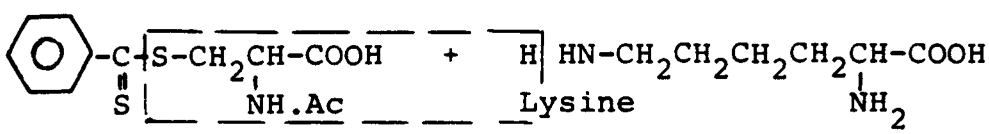

DTAC

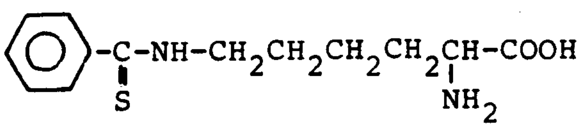

N-thiobenzoyl lysine

EXEMPLE 2

**Modification de la peroxydase de raifort au moyen de l'acide tridécyl thiomercaptoacétique $C_{12}H_{25}\underset{S}{\underset{\|}{C}}-S-CH_2COOH$ (c'est-à-dire carboxyméthyle)**

Dans 3 ml de tampon phosphate 0,1 M pH = 8,5 sont dissous 6 mg de peroxydase de raifort commerciale (Sigma type II) soit 0,15 µmole (d'après absorption à 402 nm avec $\epsilon$ = 102 $mM^{-1}.cm^{-1}$) et 1,6 mg d'acide tridécyl thiomercaptoacétique soit 5 µmoles (préalablement dissous dans 0,1 ml d'éthanol). Le mélange est agité 18 heures à température ambiante. La solution est ensuite dialysée 18 heures à 4°C contre de l'eau distillée puis lyophilisée. L'enzyme obtenue a été analysée. Elle présente les caractéristiques suivantes.

Spectre UV : apparition d'une bande à 277 nm due aux fonctions thioamide formées.

Nombre de lysines touchées : les lysines libres sont dosées par la méthode classique au TNBS (acide trinitrobenzènesulfonique), on trouve ainsi que 3 à 4 lysines ont été touchées.

Solubilités : alors que l'enzyme native n'est soluble que dans l'eau, la nouvelle enzyme est soluble à plus de 5 mg/ml dans l'éthanol, le chloroforme, le toluène, l'éther et l'eau.

Activité spécifique : il s'agit de l'activité mesurée sur l'oxydation de l'orthodianisidine par l'eau oxygénée. Un dimère est formé qui absorbe à 444nm (avec $\epsilon$ = 30 $mM^{-1}.cm^{-1}$). L'activité spécifique obtenue est 850 U par mg de protéine soit 97% de l'activité spécifique de l'enzyme native.

Activité en solvant organique : la peroxydase de raifort est connue pour catalyser la réaction d'oxydation de la 9-méthoxy éllipticine :

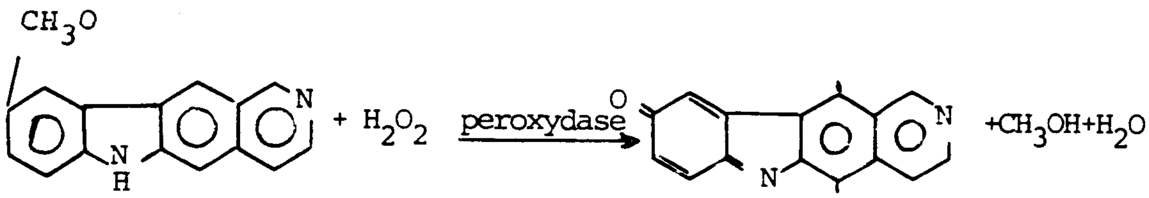


Cette réaction est intéressante car la 9-oxoellipticine est aisément réduite en 9-hydroxyellipticine par l'acide ascorbique. Le seul inconvénient réside dans la faible solubilité de la 9-méthoxyellipticine dans l'eau. La faible concentration en substrat, qui en résulte pour l'enzyme, limite la vitesse de la réaction d'oxydation.

Or, on a pu mettre en oeuvre cette réaction dans l'éther avec la peroxydase modifiée suivant l'invention. Dans une cuve de spectrophotomètre on mélange 0,5 ml de 9-méthoxyellipticine 1mM dans l'éther, 0,5 ml de peroxydase modifiée 1 mg/ml dans l'éther, 10 à 50 µl de solution aqueuse d'eau oxygénée 200 mM et on complète avec de l'éther jusqu'à un volume final de 2 ml. On suit alors à 20°C l'adsorbance à 486 nm, longueur d'onde d'absorption de la 9-oxoellipticine formée ( $\epsilon$ = 9,2 $mM^{-1}.cm^{-1}$). Plusieurs essais, effectués avec différentes quantités d'eau oxygénée, ont permis de montrer quela cinétique d'oxydation est michaelienne avec :

$K_m^H\,2^O2$ = 2 mM

$V_m$ = 0,25 µmol/mn par mg de lyophilisat de peroxydase modifiée.

## EXEMPLE 3

### Fixation de groupes thiobenzoïques sur la papaïne la rendant soluble et active dans du diméthyl sulfoxyde

Dans 40 ml de tampon phosphate 0,1 M pH = 8,5 sont dissous 0,32 g de papaïne commerciale (Sigma Type IV) et 100 mg d'acide thiobenzoyl-mercapto-acétique, préalablement dissous dans 3 ml d'éthanol. Le mélange est laissé 2 heures à 40°C puis dialysé 16 heures à 4°C contre de l'eau, puis lyophilisé. On récupère 0,26 g soit 81% de rendement chimique. L'enzyme obtenue présente les caractéristiques suivantes.

Spectre UV : déplacement de la bande d'absorption de 278 nm à 280 nm.

Nombre de lysines touchées : 2 sur les 10 que comprend la papaïne.

Solubilité : soluble dans le benzène, le DMSO et l'eau à raison de 10 mg/ml, tandis que l'enzyme native n'est pas soluble dans le benzène, seulement dansl'eau et le DMSO.

Activité amidase : c'est celle de l'hydrolyse, à pH = 7,5 et à 50°C en tampon tris et en présence de cystéine et d'EDTA, de la N-benzoyl D, L arginine para-nitroanilide (BAPA) qui libère la para-nitroaniline absorbant à 410 nm (avec $\epsilon$ = 8,8 $mM^{-1}.cm^{-1}$). Alors que l'enzyme native a une activité de 28 mU par mg de lyophilisat, l'enzyme modifiée a 11 mU par mg de lyophilisat, soit 32% de l'activité initiale ; malgré cela l'enzyme modifiée est très intéressante, parce qu'elle présente une activité en solvant organique ; on peut donc l'utiliser dans du DMSO, en l'absence d'eau,pour de la synthèse peptidique. Le DMSO convient particulièrement bien pour solubiliser de nombreux peptides, mais l'enzyme native n'a aucune activité dans ce solvant : par contre, l'enzyme modifiée présente une activité de 1,2 mU par mg de lyophilisat.

EXEMPLE 4

Modification de la papaïne par la fixation de groupes thiopropionate d'éthyle sur sa molécule

On fait réagir, en milieu aqueux le dithioester carboxyméthyl sulfure-3 dithiopropionate d'éthyle

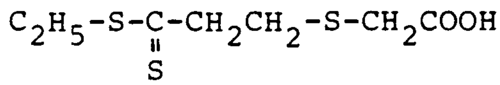

sur la papaïne.

Pour cela, dans 4,5 ml de tampon phosphate 0,1 M pH = 8,5 on mélange 36 mg de papaïne commerciale (Sigma Type IV) avec 23 mg de dithioester préalablement dissous dans 0,1 ml d'éthanol. Le mélange est porté à 40 °C pendant 2 heures, puis dialysé 16 heures à 4 °C contre du tampon phosphate 0,1 M pH = 7,2. La solution protéique obtenue est analysée et l'on trouve :

Spectre UV : déplacement maximum d'absorption de 278 nm à 270 nm.

Nombre de lysines touchées : 4,5 sur 10.

Activité enzymatique dans l'eau, perte de 50%. Les sites de la molécule modifiée peuvent être représentés, conformément à la formule (2), par

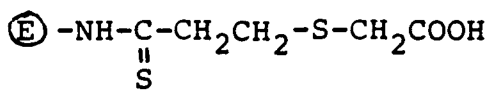

Cet exemple montre que l'on peut remplacer un groupement $-\overset{+}{N}H_3$ par un groupement $-COO^-$ ce qui permet de changer le point isoélectrique et le domaine optimum de pH de l'enzyme modifiée.

EXEMPLE 5

Fixation d'un polyméthylène glycol bis-dithioester (PEG-dithioester) sur la peroxydase de raifort

On prépare un polymère fonctionnalisé de la façon suivante. Dans 10 ml d'eau distillée, on dissout 0,25 g de PEG 20000 bis-aminé,commercial (Sigma), correspondant à 25 µmoles de fonctions-$NH_2$ libres ; on ajoute 0,4 ml de triéthylamine et 44 mg de tétrathiooctanedioate de carboxyméthyl,

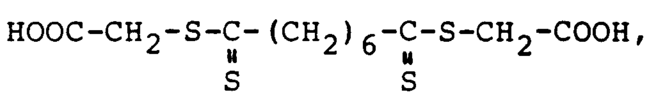

préalablement dissous dans 0,1 ml d'éthanol ; cela représente 250 µmoles de fonction dithioester. Le pH se fixe à 10,8. On laisse agiter 48 heures à la température ambiante. La solution est ensuite dialysée 16 heures à température ambiante contre de l'eau distillée. On récupère alors 13 ml d'une solution de pH = 6,6 qui est analysée.

Le spectre UV permet de doser à 308 nm les fonctions dithioester greffées (avec $\epsilon$ = 12,2 $mM^{-1}.cm^{-1}$), et à 263 nm les fonctions thioamide formées ( $\epsilon$ = 12,8 $mM^{-1}.cm^{-1}$). Ayant travaillé avec un excès de fonctions dithioesters, on peut admettre qu'il ne reste plus de fonctions $-NH_2$ libres. On a donc un produit de formule :

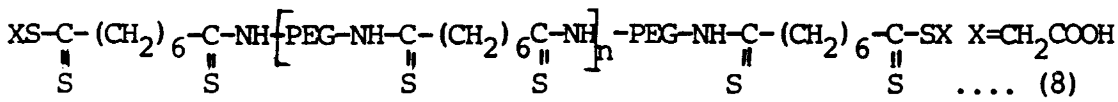
.... (8)

le rapport des concentrations en fonction thioamide (1,31 mM) et dithioester (0,18 mM) permet de calculer le degré de polymérisation n ; ce rapport vaut :

$$r = \frac{2n + 2}{2} = n + 1$$

expérimentalement on obtient :

$$r = \frac{1,31}{0,18} = 7,3 \text{ soit } n = 6,3$$

6 mg de peroxydase de raifort commerciale (Sigma Type II) sont dissous dans la solution précédemment préparée (0,15 µmole de peroxydase et 2,3 µmole de dithioester) et le pH est porté à 8,5 par dissolution de $Na_2HPO_4$ dans le mélange. On laisse agiter 18 heures à la température ambiante, puis la solution est dialysée et enfin lyophilisée. L'enzyme obtenue présente les caractéristiques suivantes.

Spectre UV : apparition d'une bande à 287 nm due aux fonctions thioamide.

Nombre de lysines touchées : 2.

Solubilité : l'enzyme est soluble à plus de 5 mg/ml dans les solvants : acétone, chloroforme, dioxanne, toluène, diméthylformamide, eau.

Activité spécifique : 780 U par mg de protéines soit 89% de l'activité spécifique de l'enzyme native.

Activité dans le chloroforme : pour l'oxydation de la 9-méthoxyellipticine, l'activité est satisfaisante. Dans une cuve de spectrophotomètre, on mélange : 0,5 ml de 9-méthoxyellipticine 1 mM dans le chloroforme, 0,3 ml de peroxydase modifiée à 1 mg/ml dans le chloroforme, 10 à 40 µl d'eau oxygénée 200 mM (en solution aqueuse) et le volume est porté à 1,5 ml avec du chloroforme. Pour différentes quantités d'eau oxygénée, on observe une cinétique michaelienne avec :

| | |
|---|---|
| $K_m^H\ 2^O2$ | : 6,25 mM |
| $V_m$ | : 83 nmoles/minute par mg de lyophilisat de peroxydase modifiée. |

## EXEMPLE 6

### Modification de la papaïne à l'aide d'un polyéthylène glycol bis-dithioester

Le polymère fonctionnalisé a été préparé comme à l'exemple 5, mais dans des conditions de plus grande dilution. Les mêmes quantités de produit ont été utilisées dans 100 ml d'eau distillée au lieu des 10 ml à l'exemple 5. On a obtenu alors un polymère moins lourd, puisque le dosage UV conduit à n = 0. On était donc en présence de

$$HOOC-H_2CS-\underset{\underset{S}{\|}}{C}-(CH_2)_6-\underset{\underset{S}{\|}}{C}-NH-PEG-NH-\underset{\underset{S}{\|}}{C}-(CH_2)_6-\underset{\underset{S}{\|}}{C}-SH_2COOH \quad ........ (9)$$

1,9 mg de papaïne commerciale (Sigma type IV) et 89 mg de $Na_2HPO_4$ sont dissous dans 23 ml de la solution susindiquée de polymère fonctionnalisé. Le volume est porté à 50 ml avec de l'eau distillée. Le pH obtenu est 8,5. La solution est portée à 40°C pendant 2 heures puis dialysée 16 heures à 4°C contre de l'eau distillée. La solution dialysée a un pH = 6,6 et un volume de 58 ml. Après lyophilisation on récupère 45 mg, soit un rendement chimique de 76%. L'analyse de l'enzyme ainsi modifiée donne les résultats que voici.

Spectre UV : la bande à 278 nm est déplacée à 269 nm avec un épaulement à 308 nm montrant que toutes les fonctions dithioesters n'ont pas réagi.

Nombre de lysines touchées : 5 lysines sur 10.

Solubilité : l'enzyme est soluble à 10 mg/ml dans le chloroforme, le DMSO et l'eau.

Activité amidase : 1,5 mU par mg de lyophilisat soit un rendement en activité :

$$\frac{45 \times 1,5}{1,9 \times 28} = 125\%$$

Activité dans le DMSO par le même test que dans l'exemple 3 : 3,0 mU par mg de lyophilisat. Dans le chloroforme, la solution de substrat est préparée dans un mélange de 80% de chloroforme avec 20% de DMSO pour solubiliser les ingrédients nécessaires au test. On trouve une activité de 4,2 mU par mg de lyophilisat. Par contre,l'enzyme native, mise en suspension (car insoluble dans le chloroforme), dans un test identique, ne donne aucune activité.

Les deux exemples 5 et 6 montrent que la modification avec PEG-dithioesters permet d'avoir de bonnes activités dans le chloroforme, ce qui apporte un progrès technique appréciable.

EXEMPLE 7

Modification d'une hémoprotéine par de l'acide dithiopentanoïque

La protéine traitée est la peroxydase de raifort de point isoélectrique 7,2.

Dans 5 ml de tampon phosphate 0,2 M pH 6,0, on dissout 25 mg de peroxydase de raifort (Sigma Type II) et 2 mg de dithioacide pur $CH_3(CH_2)_3CSSH$, ce qui correspond à des concentrations de 0,05 mM d'enzyme pour 3 mM de dithioacide.

On laisse agiter 16 heures à température ambiante. La solution est ensuite dialysée pendant 16 heures contre du tampon citrate 5 mM pH 6,5.

Le dialysat est ensuite récupéré et analysé :

Le rendement chimique global est calculé par la comparaison du nombre de moles d'hémoprotéine (déterminé par l'absorbance à 402 nm où $\epsilon = 102\ mM^{-1}.cm^{-1}$) utilisé au départ, avec celui de moles récupérées après modification. Le rendement est de 100%. Concernant l'activité, on détermine pour chaque solution de peroxydase le rapport de l'activité de la solution (en U/ml) par la concentration en hémoprotéine (en nmole/ml) ce qui conduit à l'activité spécifique,en U par nmole d'hème.

L'activité enzymatique de la solution est mesurée à 30 °C dans une cuve en plastique remplie par 1,5 ml de tampon citrate 0,1 M pH 3,5, 20 µl d'eau oxygénée 0,2 M, 20 µl d'o-dianisidine 13 mM et 20 µl de solution enzymatique préalablement diluée 20 fois. Le produit d'oxydation de l'o-dianisidine est suivi à 444 nm avec $\epsilon = 30\ mM^{-1}.cm^{-1}$. Dans le présent exemple, on passe d'une activité spécifique de 125 U/nmole d'hème pour l'enzyme native à 67 U/nmole d'hème pour l'enzyme modifiée soit 54% de rendement: puisque le rendement chimique est 100%, le rendement global en activité est aussi de 54%. Mais en revanche,l'enzyme modifiée présente l'avantage de présenter une activité ligninase de 1,4 U/µmol d'hème,alors que l'enzyme native ne la possède pas.

Le nombre de groupes lysine est déterminé par la méthode au TNBS (acide trinitrobenzène-sulfonique) : on trouve que 3 lysines sur 6 ont été modifiées par l'acide dithiopentanoïque.

EXEMPLE 8

Modification de la peroxydase de raifort par un sel de l'acide dithiobenzoïque

Dans 100 ml de tampon citrate 0,1 M pH 5,0 on dissout 2,6 mg de peroxydase de raifort (Sigma Type II) et 1,2 mg de dithiobenzoate de tetraméthylammonium

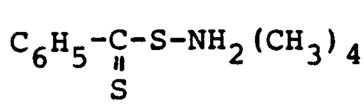

Les concentrations initiales sont ainsi de 0,26 µM en enzyme et 5,3 µM en sel du dithioacide. Après 2 heures d'agitation à température ambiante, la solution est dialysée pendant 16 heures, contre du tampon citrate 5 mM pH 6,0.

Les analyses effectuées comme à l'exemple 1 donnent :

- rendement chimique 72%
- rendement global en activité 110%
- nombre de groupes lysine greffée : 6.

On voit que le traitement suivant l'invention a accru l'activité de l'enzyme et que des thiocarbonyles se sont fixés sur tous les groupes lysine présents.

EXEMPLE 9

Modification de la lignine peroxydase de Phanerochaete Chrysosporium,par de l'acide dithiopentanoïque, $CH_3(CH_2)_3CSSH$

La protéine utilisée présente un point isoélectrique de 3,9.

A 80 ml de tampon phosphate 0,2 M pH 6,0 sont ajoutés 20 ml de préparation brute de lignine peroxydase issue d'une culture de Phanerochaete Chrysosporium (décrite dans : TIEN, M. et KIRK, T.K., 1984, Proc. Natl. Acad. Sci. US. 81, 2280-2284) et 1,5 µl de dithioacide pur. Les concentrations en hémoprotéine et dithioacide sont ainsi de 0,52 µM et 130 µM respectivement.

Le mélange est laissé en agitation pendant 7 heures à la température ordinaire, puis dialysé pendant 40 heures contre du tampon bis-tris 1 mM pH 6,0.

Les analyses sont effectuées comme dans les exemples précédents 7 et 8:

- rendement chimique, déterminé par les absorbances à 404 nm ( $\epsilon$ = 102 $mM^{-1}.cm^{-1}$), après modification, 73% par rapport à l'enzyme de départ.
- rendement global en activité : 65% ; l'activité fut déterminée à 30 °C dans une cuvette en plastique contenant 2 ml de tampon lactate 0,1 M pH 3,0, 0,1 ml d'eau oxygénée 10 mM, 0,1 ml d'alcool vératrilique 12 mM et 0,1 ml de préparation enzymatique, l'apparition d'aldéhyde vératrilique

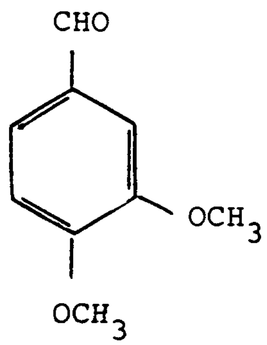

étant suivie à 310 nm prenant $\epsilon$ = 9,1 $mM^{-1}.cm^{-1}$. La légère baisse de l'activité de l'enzyme est compensée par le fait que la protéine modifiée peut être lyophilisée, avec un rendement en activité de 23%, alors que l'enzyme native perd toute son activité par lyophilisation. Le lyophilisat de l'enzyme modifiée est soluble dans du chloroforme et dans du DMSO.

EXEMPLE 10

Etude de l'effet du pH sur la modification de la lignine peroxydase

On opère avec la même enzyme qu'à l'exemple 9 et avec le même acide dithiopentanoïque.

A 80 ml de tampon citrate-phosphate 0,1 M de pH 3,0, 4,0, 5,0, 6,0 ou 6,8 on ajoute 20 ml de préparation brute de lignine peroxydase et 2 µl d'acide dithiopentanoïque. Après 3 h1/2 d'agitation à température ambiante le mélange est dialysé contre du tampon citrate-phosphate 5 mM pH 6,0 pendant 18 heures.

Les résultats en fonction du pH sont donnés dans le tableau suivant :

| pH | Rendement chimique (%) | Rendement global en activité (%) |
|---|---|---|
| 3,0 | 25 | 1 |
| 4,0 | 43 | 60 |
| 5,0 | 70 | 115 |
| 6,0 | 56 | 85 |
| 6,8 | 51 | 45 |
| 8,5 | 90 | 0 |

Pour tous les essais on observe l'absence totale de groupes lysine libres, après modification par le dithioacide. Il apparaît donc que pH 5,0 est optimal pour cette modification de la lignine peroxydase. A ce pH l'activité de l'enzyme est augmentée par le greffage de groupes $CH_3(CH_2)CS$-. Il est frappant de voir que, si l'on travaille avec un pH de 8,5, comme dans les exemples 2 à 4, l'enzyme perd son activité avec le dithiocomposé employé ici.

EXEMPLE 11

Modifications de la papaïne par de l'acide dithiopentanoïque. Effet du pH.

Le point isoélectrique de la protéase papaïne est de 8,8. Dans 4,5 ml de tampon phosphate 0,1 M pH 6,0, 6,5 ou 8,5 sont dissous 36 mg de papaïne (Sigma Type IV) et 23mg de dithioacide. Le mélange est chauffé à 40 °C pendant 2 heures puis dialysé à 4 °C pendant 16 heures contre de l'eau distillée. Les résultats en fonction du pH sont :

| pH | Rendement en activité (%) | nombre de lysines touchées |
|---|---|---|
| 6,0 | 100 | 6 |
| 6,5 | 92 | 3 |
| 8,5 | 113 | 0 |

Il apparaît clairement que les pH plus faibles favorisent la réaction de modification. D'autre part, cet exemple est important, car la papaïne est une enzyme à cystéine active. Puisque les activités ne sont pratiquement pas affectées par la modification au dithioacide, on peut en conclure que la cystéine du site actif de la protéase n'est pas atteinte par le dithioacide dans les conditions opératoires, alors que les lysines le sont.

EXEMPLE 12

Modification de la lignine peroxydase de Phanerochaete Chrysosporium par un sel de l'acide dithiobenzoïque

La protéine est la même qu'à l'exemple 9,de point isoélectrique 3,9.

On mélange 80 ml de tampon citrate 0,1 M pH 5,0 et 20 ml de préparation brute, dialysée, de lignine peroxydase.On ajoute ensuite 1,2 mg de dithiobenzoate de tétraméthylammonium. Les concentrations initiales en hémoprotéine et sel de dithioacide sont ainsi de 0,46 $\mu$M et 5,3 $\mu$M respectivement. Après 2 heures d'agitation à température ambiante, le mélange est dialysé à 4 °C pendant 16 heures contre du tampon citrate 5 mM pH 6,0. Le dialysat est analysé comme décrit à l'exemple 3, ce qui donne :

- rendement chimique 100%
- rendement global en activité 86%
- plus aucune lysine libre après modification.

Bien que l'activité soit un peu baissée, l'enzyme greffée comporte l'avantage d'être particulièrement stable ; tandis que l'enzyme native a perdu 2 à 5% de son activité par mois, conservée dans les mêmes conditions, l'enzyme modifiée a gardé 100% de son activité après 2 mois.

EXEMPLE 13

Comparatif

Le greffage de la papaïne (point isoélectrique 8,8) est effectué comme dans l'exemple 6, mais à pH 6 au lieu de 8,5 ; on constate alors que le dithioester employé ne se fixe pas sur la protéine. Par contre, comme vu dans l'exemple 11 ci-dessus, l'acide dithioïque s'y fixe sur tous les groupes lysine à pH 6.

**Revendications**

**1.** Procédé de modification d'un protide par fixation d'un groupe organique sur sa molécule, caractérisé en ce que l'on fait réagir le protide avec un composé dithoïque répondant à la formule

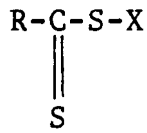

où R désigne le groupe organique à fixer, par l'intermédiaire de C = S, sur le protide, tandis que X est un groupement éliminable par la réaction de -S-X avec un reste d'amine du protide.

**2.** Procédé suivant la revendication 1, caractérisé en ce que R est un radical hydrocarboné, aliphatique ou arylique, notamment alkyle en $C_1$ à $C_{20}$, alcényle en $C_2$ à $C_{20}$, phényle, phénylényle, naphtyle, naphtylényle, alkylaryle ou aryl-alkyle.

**3.** Procédé suivant la revendication 1, caractérisé en ce que R est un groupe organique, hydrophile, polaire, négatif, positif ou neutre, en particulier porteur d'un carboxyle, hydroxyle, halogène, sulfinyle, sulfonyle, phosphoryle, phosphonyle, sulfhydryle ou amide.

**4.** Procédé suivant la revendication 1, caractérisé en ce que R est une chaîne polymère ou oligomère, en particulier polyéthylène, polypropylène, polyacrylique, polyéthylène glycol ou alcool polyvinylique.

**5.** Procédé suivant la revendication 1, caractérisé en ce que R est du type

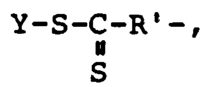

R' désignant un groupe hydrocarboné divalent, en particulier un alkylène en $C_1$ à $C_{20}$, ou un cycloalkylène, arylène ou alkyl-arylène en $C_6$ à $C_{16}$, tandis que Y est un groupement éliminable par la réaction de -S-Y avec un reste d'amine du protide.

**6.** Procédé suivant une des revendications 1 à 5, caractérisé en ce que X ou/et Y, semblables ou différents, sont constitués par une partie hydrocarbonée $(-CH_2)_n$, où n est 1 à 6, portant une fonction halogène, carboxyle, sulfinique, sulfonique, phosphoreuse,phosphoneuse, phosphorique, phosphonique, H actif, SH, amide ou hydroxy.

**7.** Procédé suivant une des revendications 1 à 5, caractérisé en ce que X est un atome d'hydrogène ou un cation provenant d'une base faible.

**8.** Procédé suivant une des revendications 1 à 7, caractérisé en ce qu'il est réalisé au sein d'un solvant aqueux, organique ou hydro-organique, ou bien en milieu hétérogène, entre 0° et 70°C, de préférence 20°-45°C, le pH du milieu étant compris entre 3 et 11,5, de préférence entre 4 et 9, notamment dans le cas d'enzymes.

**9.** Procédé suivant la revendication 8, caractérisé en ce que le pH du milieu réactionnel est de 7,0 à 11,5 lorsque le composé dithioïque est un dithioester, et de 3 à 7 quand ce composé est un acide dithioïque ou un sel de base faible d'un tel acide.

**10.** Protide modifié chimiquement, caractérisé en ce qu'au moins un de ses atomes d'azote porte un groupe

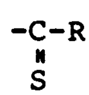

où R est un radical organique, notamment hydrocarboné, pouvant comprendre une partie polaire, positive, négative ou neutre, ou bien est constitué par une chaîne polymère.

**11.** Protide suivant la revendication 10, constitué par une enzyme caractérisée en ce qu'elle est soluble dans des solvants organiques.

**12.** Protide suivant la revendication 11, en particulier protéase ou peroxydase, soluble dans du benzène, toluène, éther, éthanol, chloroforme ou/et diméthyl sulfoxyde, dont plusieurs motifs lysine portent, par l'intermédiaire d'un

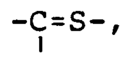

un groupe R constitué par un radical aliphatique en $C_1$ à $C_{20}$, un radical arylique en $C_6$ à $C_{16}$ ou une chaîne polymère, en particulier de polyéthylène glycol.

**13.** Protide suivant la revendication 11, caractérisé en ce qu'il est constitué par une enzyme, notamment hydrolase, susceptible de catalyser une réaction inverse, en particulier synthèse d'esters, d'amides, de thioamides et peptides.

## Claims

**1.** Process of modification of a protide by fixation of a organic group to its molecule, characterised in that the protide is reacted with a dithioic compound corresponding to the formula

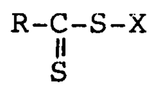

where R designates the organic group to be fixed, through the intermediary of C=S to the protide, while X is a group eliminatable by the reaction of -S-X with an amine residue of the protide.

**2.** Process according to claim 1, characterised in that R is an aliphatic or aryl hydrocarbon radical, particularly a $C_1$ to $C_{20}$ alkyl, $C_2$ to $C_{20}$ alkenyl, phenyl, phenylenyl, naphthyl, naphthylenyl, alkylaryl or aryl-alkyl.

**3.** Process according to claim 1, characterised in that R is a negative, positive or neutral polar hydrophilic organic group, in particular one carrying a carboxyl, hydroxyl, halogen, sulphinyl, sulphonyl, phosphoryl, phosphonyl, sulphydryl or amide.

**4.** Process according to claim 2, characterised in that R is a polymer or oligomer chain, in particular polyethylene, polypropylene, polyacrylic, polyethylene glycol or polyvinyl alcohol.

**5.** Process according to claim 1, characterised in that R is of the type

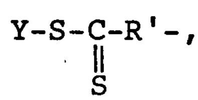

R' designating a divalent hydrocarbon group, in particular a $C_1$ to $C_{20}$ alkylene or a $C_6$ to $C_{16}$ cycloalkylene, arylene or alkyl-arylene, while Y is a group eliminatable by the reaction of -S-Y with an amine residue of the protide.

**6.** Process according to any of claims 1 to 5, characterised in that X and/or Y are the same or different and are constituted by a hydrocarbon part $(-CH_2-)_n$, where n is 1 to 6, carrying a halogen, carboxyl, sulphinic, sulphonic, phosphorous, phosphonous, phosphoric, phosphonic, active H, SH, amide or hydroxy function.

**7.** Process according to any of claims 1 to 5, characterised in that X is a hydrogen atom or a cation derived from a weak base.

**8.** Process according to any of claims 1 to 7, characterised in that it is carried out in an aqueous, organic or hydro-organic solvent or in a heterogeneous medium, between 0° and 70°C, preferably from 20° to 45°C, the pH of the medium ranging from 3 to 11.5 and preferably from 4 to 9, particularly in the case of enzymes.

**9.** Process according to claim 8, characterised in that the pH of the reaction medium is from 7.0 to 11.5 when the dithioic compound is a dithioester and from 3 to 7 when the compound is a dithioic acid or a salt of a weak base of such an acid.

**10.** Chemically modified protide, characterised in that at least one of its nitrogen atoms carries a group

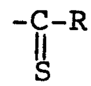

where R is an organic radical, particularly a hydrocarbon, which can comprise a positive, negative or neutral polar part, or comprises a polymer chain.

**11.** Protide according to claim 10, constituted by an enzyme characterised in that it is soluble in organic solvents.

**12.** Protide according to claim 11, in particular protease or peroxydase, soluble in benzene, toluene, ether, ethanol, chloroform and/or dimethyl sulphoxide, several lysine groups of which carry, through the intermediary of a

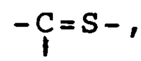

a group R comprising a $C_1$ to $C_{20}$ aliphatic radical, a $C_6$ to $C_{16}$ aryl radical or a polymer chain, in particular of polyethylene glycol.

**13.** Protide according to claim 11, characterised in that it comprises an enzyme, particularly hydrolase, capable of catalysing an inverse reaction in particular the synthesis of esters, amides, thioamides and peptides.

**Patentansprüche**

**1.** Verfahren zur Modifizierung einer stickstoffhaltigen Substanz durch Fixieren einer organischen Gruppe an ihrem Molekül, dadurch gekennzeichnet, daß man die stickstoffhaltige Substanz mit einer Dithioverbindung der Formel

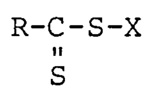

umsetzt, worin R die, über das C=S, an der stickstoffhaltigen Verbindung zu fixierende organische Gruppe bezeichnet, während X eine durch Reaktion des -S-X mit einem Aminrest der stickstoffhaltigen Verbindung eliminierbare Gruppe ist.

**2.** Verfahren nach Anspruch 1, dadurch gekennzeichnet, daß R ein aliphatischer oder aromatischer Kohlenwasserstoffrest ist, insbesondere ein $C_1$- bis $C_{20}$-Alkyl, $C_2$- bis $C_{20}$-Alkenyl, Phenyl, Phenylenyl, Naphthyl, Naphthylenyl, Alkylaryl oder Arylalkyl.

**3.** Verfahren nach Anspruch 1, dadurch gekennzeichnet, daß R eine hydrophile, polare, negative, positive oder neutrale organische Gruppe ist, die insbesondere ein Carboxyl, Hydroxyl, Halogen, Sulfinyl, Sulfonyl, Phosphoryl, Phosphonyl, Sulfhydryl oder Amid aufweist.

**4.** Verfahren nach Anspruch 1, dadurch gekennzeichnet, daß R eine polymere oder oligomere Kette ist, insbesondere eine Polyethylen-, Polypropylen-, Polyacryl-, Polyethylenglykol- oder Polyvinylalkoholkette.

**5.** Verfahren nach Anspruch 1 , dadurch gekennzeichnet, daß R vom Typ

$$Y-S-\underset{\underset{S}{\|}}{C}-R'-$$

ist,
worin R' eine divalente Kohlenwasserstoffgruppe bezeichnet, insbesondere ein $C_1$- bis $C_{20}$-Alkylen oder ein $C_6$- bis $C_{16}$-Cycloalkylen, -Arylen oder -Alkylarylen, während Y eine durch Reaktion des -S-Y mit einem Aminrest der stickstoffhaltigen Verbindung eliminierbare Gruppe ist.

**6.** Verfahren nach einem der Ansprüche 1 bis 5, dadurch gekennzeichnet, daß X und/oder Y, gleich oder verschieden, aus einem Kohlenwasserstoffteil $(-CH_2)_n$ bestehen, worin n 1 bis 6 ist, der eine Halogen-, Carboxyl-, Sulfin-, Sulfon-, Phosphor-III-, Phosphon-III-, Phosphor-V-, Phosphon-V-, aktive H-, SH-, Amid- oder Hydroxyfunktion aufweist.

**7.** Verfahren nach einem der Ansprüche 1 bis 5, dadurch gekennzeichnet, daß X ein Wasserstoffatom oder ein von einer schwachen Base abgeleitetes Kation ist.

**8.** Verfahren nach einem der Ansprüche 1 bis 7, dadurch gekennzeichnet, daß es in einem wässrigen, organischen oder wässrig-organischen Lösungsmittel oder auch in einem heterogenen Milieu zwischen 0° und 70°C, vorzugsweise 20° bis 45°C durchgeführt wird, wobei das pH des Milieus zwischen 3 und 11,5, vorzugsweise zwischen 4 und 9, insbesondere im Fall von Enzymen, liegt.

**9.** Verfahren nach Anspruch 8, dadurch gekennzeichnet, daß das pH des Reaktionsmilieus 7,0 bis 11,5 beträgt, wenn die Dithioverbindung ein Dithioester ist, und 3 bis 7, wenn die Verbindung eine Dithiosäure oder ein Salz einer schwachen Base einer solchen Säure ist.

**10.** Chemisch modifizierte stickstoffhaltige Verbindung, dadurch gekennzeichnet, daß wenigstens eines ihrer Stickstoffatome eine Gruppe

$$-\underset{\underset{S}{\|}}{C}-R$$

aufweist, worin R ein organischer Rest ist, insbesondere ein Kohlenwasserstoffrest, der einen polaren, positiven, negativen oder neutralen Teil umfassen kann, oder von einer polymeren Kette gebildet wird.

**11.** Stickstoffhaltige Verbindung nach Anspruch 10, welche von einem Enzym gebildet wird, dadurch gekennzeichnet, daß sie in organischen Lösungsmitteln löslich ist.

**12.** Stickstoffhaltige Verbindung nach Anspruch 11, insbesondere Protease oder Peroxidase, die in Benzol, Toluol, Ether, Ethanol, Chloroform und/oder Dimethylsulfoxid löslich ist, von der mehrere Lysineinheiten, über ein

$$-\underset{|}{C}=S-,$$

eine von einem aliphatischen $C_1$- bis $C_{20}$-Rest, einem $C_6$- bis $C_{16}$-Arylrest oder einer Polymerkette,

insbesondere aus Polyethylenglykol, gebildete Gruppe R aufweisen.

**13.** Stickstoffhaltige Verbindung nach Anspruch 11, dadurch gekennzeichnet, daß sie aus einem Enzym, insbesondere Hydrolase, besteht, das zur Katalyse einer inversen Reaktion geeignet ist, insbesondere zur Synthese von Estern, Amiden, Thioamiden und Peptiden.